# EUROPEAN PATENT SPECIFICATION

(11) **EP 2 480 262 B1**
(45) Date of publication and mention of the grant of the patent: **08.01.2014**
(21) Application number: 10759780.9
(22) Date of filing: 20.09.2010
(51) Int. Cl.: A61L 31/14

(54) **SHAPE MEMORY EMBOLIC PARTICLES HAVING A HIGH PACKING FRACTION**
EMBOLISCHE FORMGEDÄCHTNISPARTIKEL MIT HOHER PACKUNGSDICHTE
PARTICULES EMBOLIQUES À MÉMOIRE DE FORME À FRACTION DE BOURRAGE ÉLEVÉE

(30) Priority: 21.09.2009 US 563303
(43) Date of publication of application: 01.08.2012
(73) Proprietor: Cook Medical Technologies LLC, Bloomington, IN 47404 (US)
(72) Inventor: TEKULVE, Kurt, J., Ellettsville, IN 47429 (US)
(74) Representative: Garratt, Peter Douglas
(86) International application number: PCT/US2010/049489
(87) International publication number: WO 2011/035240

(56) References cited:
- US-A1- 2004 091 543
- US-A1- 2004 158 185
- LAURENT ET AL: "Microspheres and Nonspherical Particles for Embolization", TECHNIQUES IN VASCULAR AND INTERVENTIONAL RADIOLOGY, XX, XX, vol. 10, no. 4, 1 December 2007 (2007-12-01), pages 248-256, XP022756928, ISSN: 1089-2516, DOI: DOI:10.1053/J.TVIR.2008.03.010 [retrieved on 2008-06-20]
- J.M. DALLAVALLE, C. ORR, JR., AND H.G. BLOCKER: "Fitting Bimodal Particle Size Distribution Curves, Comparison of methods", INDUSTRIAL AND ENGINEERING CHEMISTRY, June 1951 (1951-06), pages 1377-1380, XP002637120,

## Description

### FIELD

The present disclosure relates generally to embolic particles and methods of using such particles to occlude the flow of fluid in a body vessel. More specifically, the present disclosure relates to the use of an embolization system having a high packing fraction of embolic particles.

### BACKGROUND

Embolization may be defined as the therapeutic introduction of various substances into a body vessel to fully occlude the vessel, to arrest or prevent hemorrhaging or gastrointestinal bleeding, or to reduce blood flow to an arteriovenous malformation, such as fibroids, tumors, or aneurysms. The use of embolic particles leads to the formation of an occlusion that is not substantially absorbed by the body or easily removable therefrom.

The ability to stop the flow of fluid through the pores established in and around embolic particles that are compacted together in a body vessel is critical to ensure the complete occlusion of fluid through the body vessel. Since this fluid flow depends on diffusivity through the compacted particles, the properties associated with these particles, such as packing fraction, are important for limiting or reducing said flow. Accordingly, there exists a continual desire to provide embolic particles that will effectively compact together leading to a high particle packing fraction in order to ensure occlusion of the body vessel.

US2004/091543 discloses embolic compositions comprising particles with shape memory properties and having a collapse as well as expand state.

Laurent and Al., "Microspheres and Nonspherical Particles for Embolization", Techniques in vascular and interventional radiology, discloses microspheres and nonspherical particles for embolization having a calibrated size and comparative clinical trials.

### SUMMARY

The present disclosure provides an embolization system having a high packing fraction of embolic particles for use in occluding the flow of body fluid through a body vessel. One embodiment of an embolization system, constructed in accordance with the teachings of the present disclosure, generally comprises a mixture of embolic particles made from a shape memory material that exhibit both a collapsed state and an expanded state. The mixture of embolic particles, which is delivered into the body vessel in the collapsed state, exhibits at least a bimodal particle diameter distribution in such collapsed state. This bimodal distribution, which has a first mean diameter (D_{1C}) and a second mean diameter (D_{2C}), exhibits a ratio of D_{1C} / D_{2C} that is less than about 1 / 7. The mixture of embolic particles maintains at least a bimodal particle diameter distribution upon transitioning to its expanded state. In such an expanded state, the bimodal distribution has a first mean diameter (D_{1E}) and a second mean diameter (D_{2E}) that defines the ratio of D_{1E} / D_{2E} to be less than about 1 / 7. The embolic particle mixture is adapted such that the ratio of D_{1E} / D_{1C} and D_{2E} / D_{2C} is greater than about 1.5 with the expanded state of the mixture having a particle packing fraction in the body vessel that is at least about 0.85. The particle packing fraction of the mixture in its expanded state causes the occlusion of the flow of body fluid through the body vessel.

According to another aspect of the present disclosure, the mixture of embolic particles may include a tri-modal or quad-modal distribution of particle sizes leading to an increase in the particle packing fraction for the embolic particles when in their expanded state. The embolization system may further include some particles that are not made from a shape memory material. These particles exhibit a similar mean particle diameter in both the collapsed state and expanded state.

According to another aspect of the present disclosure, the step of preparing a mixture of embolic particles in the collapsed state may further include a particle diameter distribution that is tri-modal or quad-modal in nature. Such tri-modal or quad-modal distributions are maintained upon transitioning of the embolic particles from their collapsed state to their expanded state.

Further areas of applicability will become apparent from the description provided herein.

### BRIEF DESCRIPTION OF THE DRAWINGS

The drawings described herein are for illustration purposes only and are not intended to limit the scope of the present disclosure in any way.
Figure 1A is a graphical depiction of the volume concentration of embolic particles in an embolization system according to one embodiment of the present disclosure plotted as a function of particle diameter. This graph shows a mixture of embolic particles having a bimodal particle size distribution in the collapsed state and in the expanded state;
Figure 1B is a cross-sectional schematic representation of the embolic particles of Figure 1A in their collapsed state as delivered into the vasculature of a patient;
Figure 1C is a cross-sectional schematic representation of the embolic particles of Figure 1 B shown in their expanded state;
Figure 2A is a graphical depiction of the volume concentration of embolic particles in an embolization system according to another aspect of the present disclosure depicting a tri-modal particle size distribution in the collapsed state and in the expanded state when plotted as a function of particle diameter;
Figure 2B is a cross-sectional schematic representation of the embolic particles of Figure 2A in their collapsed state as delivered into the vasculature of a patient;
Figure 2C is a cross-sectional schematic representation of the embolic particles of Figure 2B shown in their expanded state;
Figure 3A is a graphical depiction of the volume concentration of embolic particles in an embolization system according to another aspect of the present disclosure depicting a quad-modal particle size distribution in the collapsed state and in the expanded state when plotted as a function of particle diameter;
Figure 3B is a cross-sectional schematic representation of the embolic particles of Figure 3A in their collapsed state as delivered into the vasculature of a patient;
Figure 3C is a cross-sectional schematic representation of the embolic particles of Figure 3B shown in their expanded state;
Figure 4A is a graphical depiction of the volume concentration of embolic particles in an embolization system according to yet another aspect of the present disclosure depicting a quad-modal particle size distribution in the collapsed state and in the expanded state when plotted as a function of particle diameter;
Figure 4B is a cross-sectional schematic representation of the embolic particles of Figure 4A in their collapsed state as delivered into the vasculature of a patient;
Figure 4C is a cross-sectional schematic representation of the embolic particles of Figure 4B shown in their expanded state; and
Figure 5 is a schematic depiction of a process according to another embodiment of the present disclosure for using an embolization system comprising embolic particles that exhibit a collapsed configuration for delivery into the body vessel and an expanded configuration to occlude the flow of fluid in a body vessel.

### DETAILED DESCRIPTION

The following description is merely exemplary in nature and is in no way intended to limit the present disclosure or its application or uses. It should be understood that throughout the description and drawings, corresponding reference numerals indicate like or corresponding parts and features.

The present disclosure generally provides an embolization system having a high packing fraction of embolic particles for use in occluding the flow of body fluid through a body vessel near a targeted site. Such an occlusion is useful in the treatment of a tumor, an aneurysm, or to stop the occurrence of hemorrhaging or gastrointestinal bleeding, among other reasons. For example, an aneurysm typically occurs in blood vessels at a location where the strength of the blood vessel has been compromised or weakened. In this situation, the flow of fluid through the blood vessel can cause the blood vessel wall to lose its shape, resulting in a ballooning or stretching of the blood vessel at the weakened location. If left untreated, the blood vessel wall may continue to deform until it fails, the result of which is often fatal. Embolic particles delivered to a targeted site near the aneurysm can pack together and block the flow of fluid to and around the aneurysm, thereby, reducing the chance that the aneurysm will continue to deform.

The ability to stop the flow of fluid through pores established in the packed embolic particles is critical to the complete occlusion of fluid through the body vessel. Since the fluid flow depends on diffusivity through the packed particles, many of the properties of the packed particles, such as packing fraction and pore size, may be adapted to limit or reduce such an occurrence.

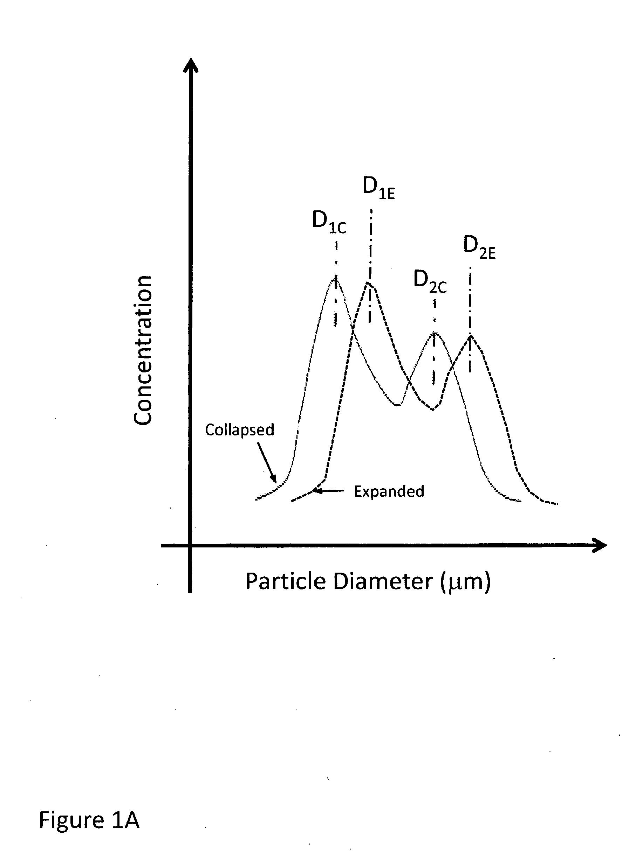

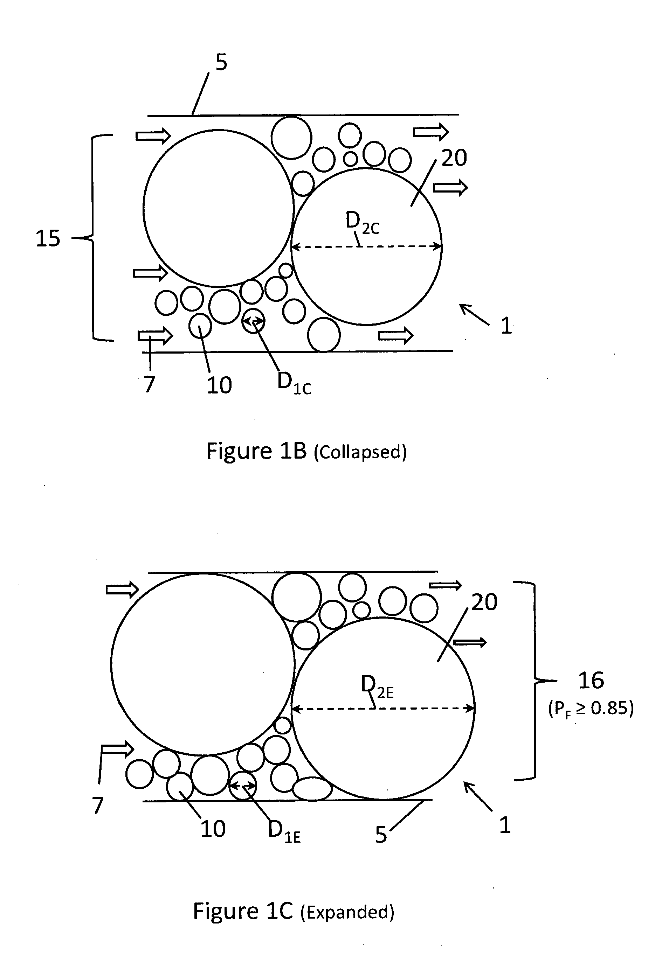

According to one embodiment of the present disclosure, the embolization system comprises a mixture of embolic particles made from a shape memory material. Referring to Figures 1A-1C, the mixture of embolic particles 10, 20 in the embolization system 1 exhibits both a collapsed state 15 and an expanded state 16. This mixture of particles 10, 20 exhibits at least a bimodal particle diameter distribution in the collapsed state 15. This bimodal distribution is defined by a first mean diameter (D_{1C}) and a second mean diameter (D_{2C}) giving rise to a ratio of D_{1C} / D_{2C} that is less than about 1 / 7. The embolic particles 10, 20 are delivered into the body vessel 5 in their collapsed state 15. Upon transitioning from their collapsed state 15 to their expanded state 16, the embolic particles 10, 20 maintain at least a bimodal particle diameter distribution. The bimodal distribution in the expanded state 16 is defined by a first mean diameter (D_{1E}) and a second mean diameter (D_{2E}) with the ratio of D_{1E} / D_{2E} being less than about 1 / 7. The embolic particles 10, 20 are adapted such that the ratio of D_{1E} / D_{1C} and D_{2E} / D_{2C} is greater than about 1.5 with the expanded state 16 of the mixture of particles having a particle packing fraction (P_{F}) in the body vessel that is at least about 0.85. The particle packing fraction of the embolic particles 10, 20 in their expanded state 16 causes the occlusion of the flow 7 of body fluid through the body vessel 5.

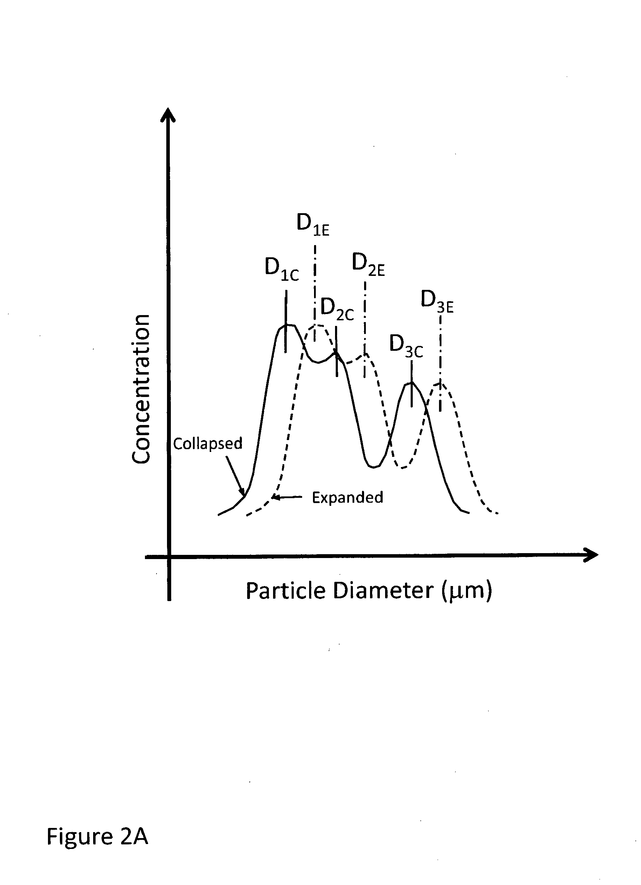

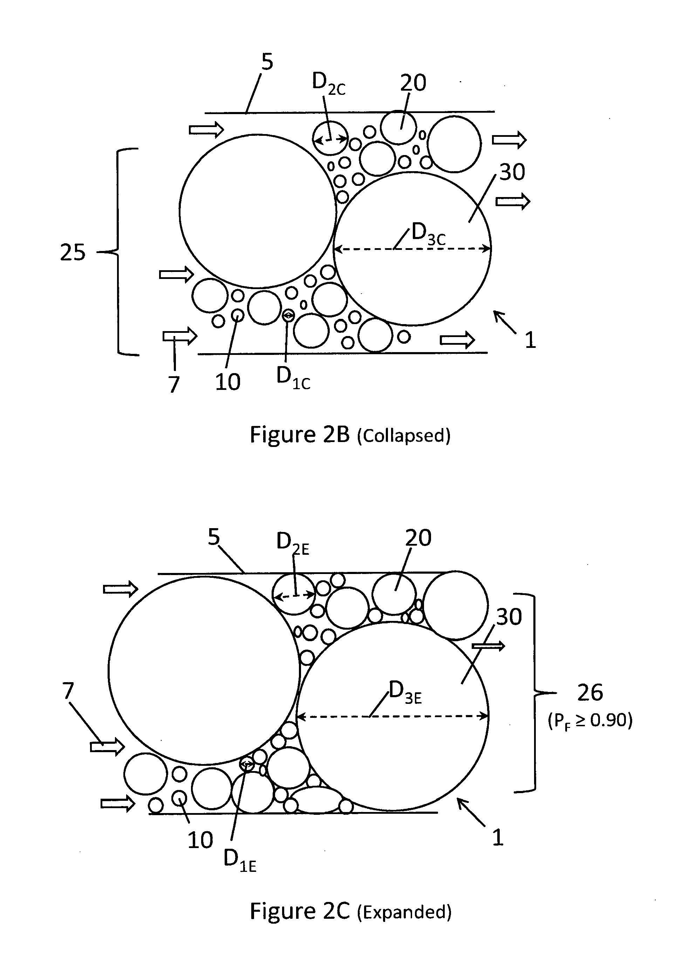

According to another aspect of the present disclosure, the embolization system 1 comprises a mixture of embolic particles 10, 20, 30 having a tri-modal particle diameter distribution as shown in Figures 2A-2C. In other words, the bimodal mixture of embolic particles 10, 20, which has a first mean diameter (D_{1C}) and a second mean diameter (D_{2C}), may further include another embolic particle 30 distribution that gives rise to a third mean diameter (D_{3C}) in a collapsed state 25. This tri-modal mixture of embolic particles 10, 20, 30 is such that the ratio of D_{2C} / D_{3C} is less than about 1 / 7. Upon transitioning from its collapsed state 25 to its expanded state 26, the mixture of embolic particles 10, 20, 30 maintains its tri-modal particle distribution in the expanded state 26. The tri-modal distribution in the expanded state 26 exhibits a first mean diameter (D_{1E}), a second mean diameter (D_{2E}), and a third mean diameter (D_{3E}). The ratio of D_{2E} / D_{3E} is less than about 1 / 7 with the particle packing fraction (P_{F}) of the mixture 10, 20, 30 in the expanded state being greater than about 0.90. The particle packing fraction of the mixture of embolic particles 10, 20, 30 in its expanded state 16 causes the occlusion of the flow 7 of body fluid through the body vessel 5.

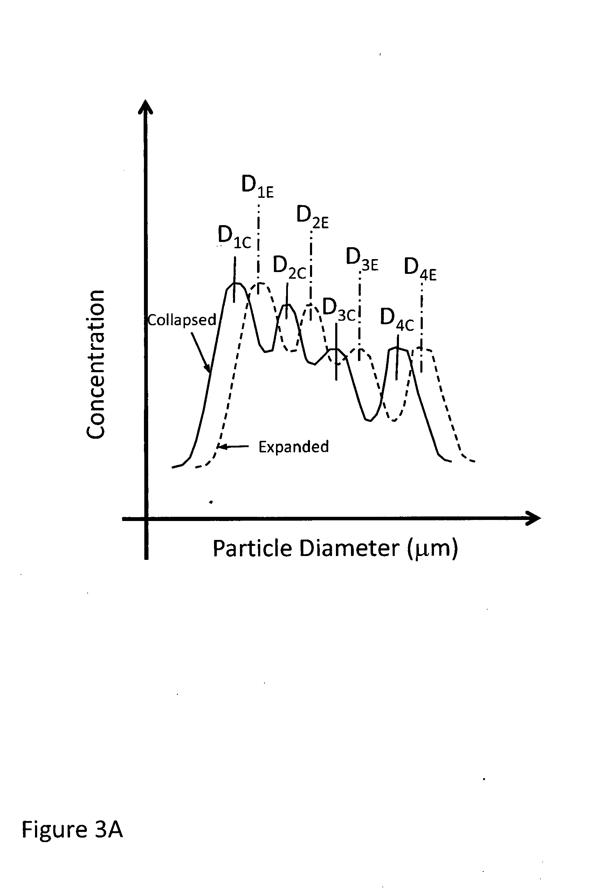

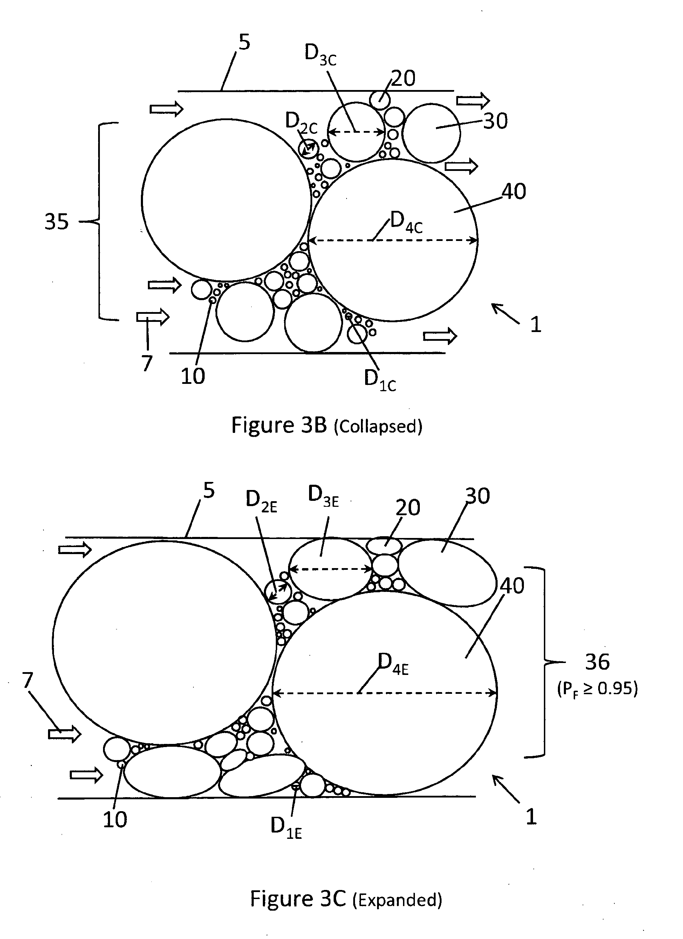

According to yet another aspect of the present disclosure, the embolization system 1 comprises a mixture of embolic particles 10, 20, 30, 40 that exhibits a quad-modal particle distribution as shown in Figures 3A-3C. In other words, the mixture of embolic particles 10, 20, 30, 40 exhibits not only a first mean diameter (D_{1C}), a second mean diameter (D_{2C}), and a third mean diameter (D_{3C}), but also a fourth mean particle diameter (D_{4C}) in the collapsed state 35, This quad-modal mixture of embolic particles 10, 20, 30, 40 is such that the ratio of D_{3C} / D_{4C} is less than about 1 / 7. Upon transitioning from its collapsed state 35 to its expanded state 36, the mixture of embolic particles 10, 20, 30, 40 maintains its quad-modal particle distribution in the expanded state 36. The quad-modal distribution in the expanded state 36 exhibits a first mean diameter (D_{1E}), a second mean diameter (D_{2E}), a third mean diameter (D_{3E}), and a fourth mean diameter (D_{4E}). The ratio of D_{3E} / D_{4E} is less than about 1 / 7 with the particle packing fraction (P_{F}) of the embolic particle mixture 10, 20, 30, 40 in the expanded state being greater than about 0.95.

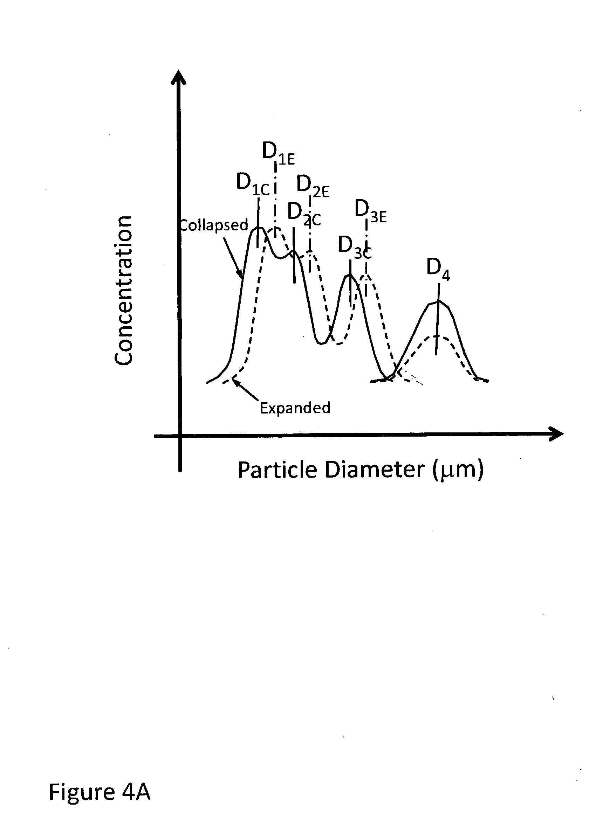

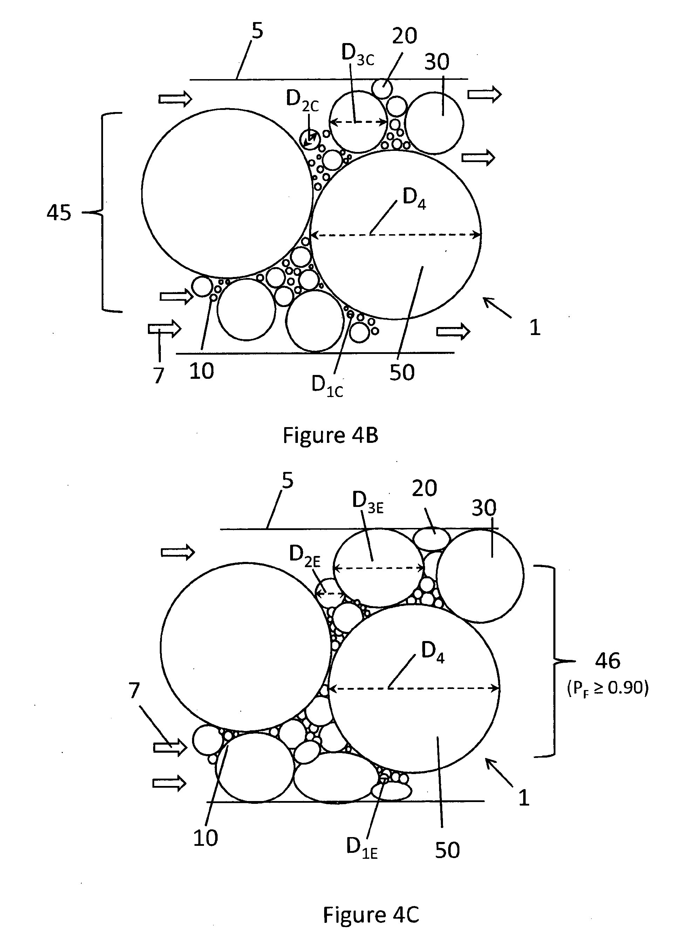

According to another aspect of the present disclosure, the embolization system 1 may comprise a combination of particles made from a shape memory material and particles that are not made from a shape memory material. In this case, the diameter of the embolic particles made from the shape memory material will increase upon transitioning from a collapsed state to an expanded state. However, the diameter of the embolic particles that are not made from a shape memory material will remain substantially the same between the collapsed state and the expanded state. For example, referring now to Figures 4A-4C, an embolization system 1 having a mixture of embolic particles 10, 20, 30 made from a shape memory material that exhibits a tri-modal particle distribution with particle diameters given in the collapsed state by D_{1C}, D_{2C}, & D_{3C} and in the expanded state of D_{1E}, D_{2E}, & D_{3E} may further incorporate particles 50 that are not made from a shape memory material, which gives rise to a fourth particle diameter distribution of D₄ in both the collapsed state and expanded state. Although this example describes an embolization system 1 that has a quad-modal particle size distribution of embolic particles 10, 20, 30, 50 with the mean particle diameter, D₄, of one of the particle distributions being unaffected by transitioning between a collapsed state to an expanded state, one skilled-in-the-art will understand that a tri-modal particle size distribution of embolic particles where one of the mean particle diameters, i.e., D₃, of one of the particle distributions is unaffected by transitioning between a collapsed state to an expanded state, may also be utilized.

The particle packing fraction (P_{F}) of the embolic particles in the embolization system 1 where all of the particles are made from a shape memory material may be greater than the particle packing fraction (P_{F}) exhibited by an embolization system 1 having a similar distribution of particle sizes in the collapsed state, but includes some particles that are not made from a shape memory material. For example, an embolization system 1 comprising a mixture of embolic particles 10, 20, 30 made entirely from a shape memory material and giving rise to a tri-modal particle size distribution will exhibit a packing fraction (P_{F}) greater than about 0.90. In comparison, an embolization system 1 comprising a mixture of embolic particles 10, 20 made from a shape memory material and embolic particles 50 that are not made from a shape memory material that also gives rise to a tri-modal particle size distribution will exhibit a packing fraction (P_{F}) greater than about 0.85. Similarly an embolization system 1 exhibiting a quad-modal particle size distribution will exhibit a particle packing fraction (P_{F}) in the expanded state of greater than about 0.95 when all particles are made from a shape memory material and greater than about 0.90 when one of the particle distributions (i.e., D₄) comprises particles that are not made from a shape memory material as shown in Figures 4A-4C.

The embolization system 1 when in its collapsed state may contain a mixture of embolic particles dispersed and suspended within a suitable, biocompatible carrier medium, such as a saline solution. A mixture of embolic particles suspended in a carrier medium can be prepared in calibrated concentrations in order to ease its delivery into the vasculature of the patient. The density of the composition may range from about 1.1 to 1.5 g/cm³ with the weight ratio of the embolic particles to the carrier medium being about 0.01 to 20% by weight. The physician may determine the concentration of embolic particles suspended in the carrier medium by adjusting the weight ratio of the embolic particles to the carrier medium. The suspension of embolic particles in a carrier medium should be stable for between about 1 to 10 minutes in order for the physician to deliver the embolic particles to the targeted site within the vasculature of the patient.

The embolization system 1 comprises embolic particles that are either entirely comprised of a shape memory material or a mixture of particles made from a shape memory material with particles that are not made from a shape memory material. Any biocompatible material known to one skilled-in-the-art may be utilized as the embolic particles that are not made from a shape memory material. Examples of biocompatible materials include polyesters, nylon, polytetrafluoroethylene (PTFE), or polypropylene, among others. The embolization system 1 is configured in a collapsed state and may expand to a predetermined shape capable of occluding the body vessel. The shape memory material induces the embolization system 1 to transition from its collapsed state to an expanded state upon exposure to a predetermined stimulus.

The embolic particles made from a shape memory material may be deformed from a memorized or unconstrained shape (expanded state) into a different collapsed shape. This collapsed shape will remain stable for a period of time before the shape memory material is activated and returns to its unconstrained shape. The process of creating a memorized shape may include, but not be limited to, deforming the shape memory material into a deformed shape and cooling it to a storage temperature. Cooling the deformed shape memory material to a storage temperature serves to keep the shape memory material in its deformed shape and to avoid activation of the shape memory material. Activation may include subjecting the shape memory material to a stimulus such as heat, radiation, pH, a chemical, or other stimuli known to one skilled-in-the-art. The shape memory material may provide a permanent occlusion, i.e., the occlusion is not substantially absorbed by the body and/or is not intended to be removed from the body when in its expanded state.

The shape memory material can be a polymer or a metal alloy. Examples of shape memory alloys include, but are not limited to, TiNi (Nitinol), CuZnAl, and FeNiAl alloys. The metal alloy may also be a superelastic or pseudo-elastic alloy known to one skilled-in-the-art. Examples of shape memory polymers include but are not limited to polyvinyl alcohol (PVA), polyethers, polyether esters, polyether amides, polyacrylates, polyamides, polysiloxanes, polyurethanes, polyurethane-ureas, and urethane-butadiene copolymers. The shape memory polymer may be cross-linked or crystalline in nature. The polymer may be set to its expanded state during the occurrence of cross-linking. The collapsed state may subsequently be formed by heating the polymer beyond its a softening point where it is compacted into the collapsed state, and subsequently cooled below its softening point to maintain the collapsed state. When the polymer is subjected to a temperature above its softening point, the polymer will transition from its collapsed state to its expanded state.

A variety of techniques can be used to form an embolic particle from a shape memory material. Examples of suitable techniques include, but are not limited to, micro- or nano-machining, nanoetching, nanoassembly, and micro-electromechanical (MEM) techniques. The embolic particles can be formed by polymer extrusion, polymer molding, or by stamping a sheet of a shape memory alloy, as well as by any other method known to one skilled-in-the-art. The particles may be sterilized prior to being packaged using, for example, electron-beam irradiation.

A complete description of particles includes a discussion regarding preferred diameters (size), shapes, and surface reactivity in regards to agglomeration, as well as the presence of surface moieties that may sterically or electrostatically affect agglomeration. In order to achieve high packing densities, it is desirable to limit the physical interaction that may occur between particles. This can be accomplished via any means known to one skilled-in-the-art, including but not limited to the following examples. First, the embolic particles may be spherical in nature in order to limit the area that will be in contact with neighboring particles. However, one skilled-in-the-art will understand that non-spherical particles may also be utilized.

Large differences in particle sizes can improve the packing density by filling the interstitial voids between embolic particles. A broad particle size distribution assists in determining a high packing fraction for the particles. When small particles are allowed to fill the voids between larger particles, a higher packing fraction will result. Spheres of a single size are known to provide a packing fraction on the order of only about 0.50 to 0.75, which means that the packing of such particles contains about 25-50% voids. Preferably, at least a bimodal distribution of particle sizes is used in the embolization system in order to increase the particle packing fraction. The ratio of the mean particle diameter for each of the particle size distributions should be on the order of about 1 : 7.

The particle size is an important variable for use in controlling the overall packing fraction or density. Preferably the largest particle diameter in its expanded state is about an order of magnitude less than the diameter of the vasculature in order to achieve a high packing fraction. Thus the largest mean particle diameter for the embolic particles in its expanded state is about 10% of the diameter for the vasculature in which the particles are delivered.

Vibration of the embolic particles during the introduction of the particles into the body vessel is preferable in order to enhance the packing of said particles. Such vibration may arise from moving the catheter or guide wire back and forth in a proximal-distal direction or a side to side direction during the delivery of the embolic particles to the targeted site or immediately thereafter.

A negative consequence related to the clustering of the embolic particles is a decrease in the packing fraction or density. The packing fraction associated with single size spheres may decrease to about 0.35 with the occurrence of particle agglomeration. Agglomeration or clustering of small particles arises due to cohesion between particles. The attractive forces that exist between particles may become larger in direct relationship to an increase in the surface area associated with the particles. In order to overcome these attractive forces, the use of additives or surface treatments that either sterically hinder the particles from coming close to one another or that electrostatically induce like charges on the surface of the particles to repel the particles from one another can be used to reduce or eliminate agglomeration. Examples of surface active agents include, but are not limited to, polyvinyl alcohol, stearic acid, sodium oleate, glycerine, and oleic acid. Generally, a surface active agent having a backbone of at least about eight carbon atoms can function to inhibit agglomeration.

The embolic particles may optionally be delivered with other materials, such as a contrast agent, a radiopaque agent, or a therapeutic agent, among others, as well as mixtures thereof. The embolic particles may be delivered along with other liquid embolic materials, for example, n-butyl cyanoacrylates (NBCA), polyvinyl alcohol (PVA) foam, or other hydrogel embolic materials. Several examples of therapeutic agents include, but are not limited to, anti-thrombogenic agents, anti-proliferative agents, anti-inflammatory agents, anti-cancer agents, anesthetic agents, anticoagulants, and vascular cell growth promoters.

Another objective of the present disclosure is to provide a method of occluding the flow of body fluid through a body vessel of a patient using the embolization system described above. In general, the method comprises the delivery of an embolization system in which the embolic particles are in their collapsed state to a targeted site in the body vessel. The embolic particles are then allowed to transition from their collapsed state to their expanded state. The transition from a collapsed state to an expanded state causes the embolic particles to compact, thereby, leading to a high packing fraction. In a collapsed configuration, the interparticle attraction between particles is dominated by weak van der Waal forces. However, upon transitioning of the particles of the present disclosure to an expanded configuration, the number of contacts and the interfacial attractive area for the embolic particles increases, thereby, increasing the packing fraction.

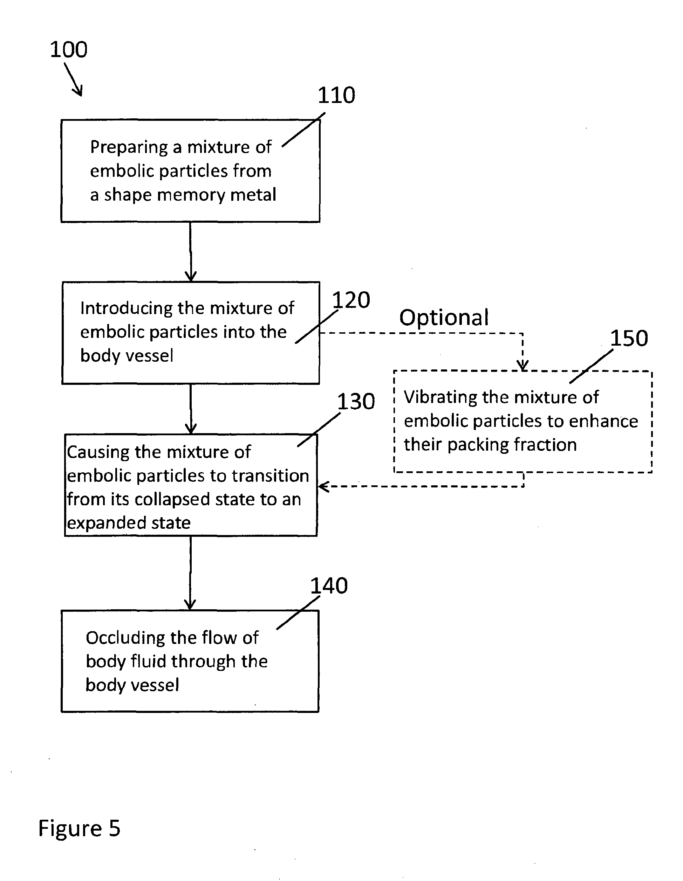

Referring now to Figure 5, the method 100 for using the embolization system 1 described herein comprises preparing 110 a mixture of embolic particles made from a shape memory material having a collapsed state and an expanded state. This mixture has at least a bimodal particle diameter distribution in the collapsed state, the bimodal distribution having a first mean diameter (D_{1C}) and a second mean diameter (D_{2C}), the ratio of D_{1C} / D_{2C} being less than about 1 / 7. The mixture of embolic particles is then delivered 120 to a targeted site in the body vessel of the patient. Then the mixture of embolic particles is caused 130 to transition from their collapsed state to their expanded state. The mixture of embolic particles maintains at least a bimodal particle diameter distribution in their expanded state. The bimodal distribution of the particles in their expanded state is defined by a first mean diameter (D_{1E}) and a second mean diameter (D_{2E}) with the ratio of D_{1E} / D_{1C} and D_{2E} / D_{2C} being greater than about 1.5, the ratio of D_{1E} / D_{2E} being about equal to the ratio D_{1C} / D_{2C}, and D_{2E} being less than about 10% of the diameter (D_{Vessel}) of the body vessel. The flow of body fluid is occluded 140 from flowing through the body vessel by the expanded state of the mixture whose particle packing fraction in the body vessel is at least about 0.85.

According to another aspect of the present disclosure, the step of preparing 120 a mixture of embolic particles in the collapsed state may further include a particle diameter distribution that is tri-modal in nature with the mixture having a third mean diameter (D_{3C}) and the ratio of D_{2C} / D_{3C} being less than about 1 / 7. In this case, the step of causing 130 the embolic particles to transition from a collapsed state to an expanded state will result in the particle diameter distribution remaining tri-modal in the expanded state, the mixture of particles having a third mean diameter (D_{3E}), and the ratio of D_{2E} / D_{3E} being less than about 1 / 7 with D_{3E} being less than about 10% the diameter of the body vessel (D_{Vessel}). The step of occluding 140 the flow of body fluid in the body vessel is then accomplished by the particle packing fraction of the mixture in the body vessel in its expanded state being greater than about 0.90.

According to another aspect of the present disclosure, the step of preparing 110 a mixture of embolic particles in the collapsed state may include a fourth mean particle diameter (D_{4C}) in the mixture, the ratio of D_{3C} / D_{4C} being less than about 1 / 7. In this case, the step of causing 130 the embolic particles to transition from a collapsed state to an expanded state results in the mixture maintaining a fourth mean diameter (D_{4E}) in the expanded state, the ratio of D_{3E} / D_{4E} being less than about 1 / 7 with D_{4E} being less than about 10% the diameter of the body vessel (D_{Vessel}). The step of occluding 140 the flow of body fluid in the body vessel is then accomplished by the particle packing fraction of the mixture in the body vessel in its expanded state being greater than about 0.95.

According to yet another aspect of the present disclosure, the step of preparing 110 a mixture of embolic particles in the collapsed state may further include particles whose diameter in the collapsed state is substantially the same as their diameter in the expanded state. In other words, embolic particles that are not made of a shape memory material may be incorporated into the embolization system.

The mixture of embolic particles may be delivered 120 to the target site in their collapsed state using a catheter placed near the intended site. The collapsed state of the embolic particles enables the suspended mixture to be capable of flowing through the catheter without aggregation. The mixture of embolic particles dispersed or suspended in a carrier medium can exhibit a temperature that is lower than the transition temperature in order to inhibit the shape memory material in the embolic particles from transitioning from their collapsed state to their expanded state. The particles once delivered to the targeted site can expand and aggregate to occlude the body vessel.

Referring once again to Figure 5, the method 100 may further include a step of vibrating 150 the embolic particles during the introduction of the particles at the targeted site in the body vessel or immediately thereafter in order to enhance the packing of said particles. Such vibratory motion may arise from moving the catheter or guidewire back and forth in a proximal-distal direction or in a side to side direction.

A person skilled-in-the-art will recognize that any measurements described herein, such as those used to determine particle size distributions and particle packing fractions, are standard measurements that can be obtained by a variety of different test methods. For example, particle size distributions may be measured using such known standard method as ASTM B822-02. Maximum particle packing fractions can be determined using standard methods, such as those described in ASTM D4512-85 or ASTM D4781-88, among others.

## Claims

1. An embolization system having a high packing fraction of embolic particles for use in occluding the flow of body fluid through a body vessel, the embolization system comprising:
mixture of embolic particles made from a shape memory material, the embolic particles having a collapsed state and an expanded state and preferably being spherical in shape ;
the mixture having at least a bimodal particle diameter distribution in the collapsed state, the bimodal distribution having a first mean diameter (D_{1C}) and a second mean diameter (D_{2C}), the ratio of D_{1C}/D_{2C} being less than about 1/7;
the mixture maintaining at least a bimodal particle diameter distribution in the expanded state, the bimodal distribution having a first mean diameter (D_{1E}) and a second mean diameter (D_{2E}), the ratio of D_{1E}/D_{2E} being less than about 1/7;
the mixture being adapted such that the ratio of D_{1E}/D_{1C} and D_{2E}/D_{2C} is greater than about 1.5 with the expanded state of the mixture having a particle packing fraction in the body vessel that is at least about 0.85;
wherein the mixture of embolic particles is delivered into the body vessel in the collapsed state, the particle packing fraction of the mixture in its expanded state causes the occlusion of the flow of body fluid through the body vessel.

2. The embolization system of Claim 1, wherein the shape memory material is one selected from the group of polyvinyl alcohol, polyurethane, polymethylmethacrylate, poly(vinyl chloride), polyethylene, metals, metal alloys, and mixtures and combinations thereof.

3. The embolization system of Claim 1, wherein the particle diameter distribution is tri-modal in the collapsed state, the mixture having a third mean diameter (D_{3C}), the ratio of D_{2C}/D_{3C} being less than about 1/7;
the particle diameter distribution remaining tri-modal in the expanded state having a third mean diameter (D_{3E}), the ratio of D_{2E}/D_{3E} being less than about 1/7 with the particle packing fraction of the mixture in the expanded state being greater than about 0.90.

4. The embolization system of Claim 3, wherein the mixture further exhibits a fourth mean particle diameter (D_{4C}) in the collapsed state, the ratio of D_{3C}/D_{4C} being less than about 1/7;
the mixture maintaining a fourth mean diameter (D_{4E}) in the expanded state, the ratio of D_{3E}/D_{4E} being less than about 1/7 with the particle packing fraction of the mixture in the expanded state being greater than about 0.95.

5. The embolization system of Claim 1, wherein the mean particle diameter of the embolic particles in the expanded state is less than about 10% of the diameter of the body vessel.

6. The embolization system of Claim 1, wherein the particle diameter distribution is tri-modal in the collapsed state, the mixture having a third mean diameter (D₃), the ratio of D_{2C}/D₃ being less than about 1/7;
the particle diameter distribution remaining tri-modal in the expanded state the third mean diameter (D₃) remaining unchanged from its diameter exhibited in the collapsed state, the particle packing fraction of the mixture in the expanded state being greater than about 0.85.

7. The embolization system of Claim 3, wherein the mixture further exhibits a fourth mean particle diameter (D₄) in the collapsed state, the ratio of D₃/D₄ being less than about 1/7;
the mixture maintaining a fourth mean diameter (D₄) in the expanded state that is unchanged from its diameter exhibited in the collapsed state, the particle packing fraction of the mixture in the expanded state being greater than about 0.90.

8. The embolization system of Claim 1, wherein the embolization system further comprises one selected from the group of a contrast agent, a radiopaque agent, a therapeutic agent, liquid embolic materials, and mixtures thereof.

9. A method of providing an embolization system for occluding the flow of body fluid through a body vessel of a patient, the method comprising
preparing a mixture of embolic particles made from a shape memory material having a collapsed state and an expanded state and preferably being spherical in shape, the mixture having at least a bimodal particle diameter distribution in the collapsed state, the bimodal distribution having a first mean diameter (D_{1C}) and a second mean diameter (D_{2C}), the ratio of D_{1C}/D_{2C} being less than about 1/7;
which mixture of embolic particles is:
adapted for delivery to a targeted site in the body vessel of the patient;
configured to transition from the collapsed state to an expanded state; the mixture maintaining at least a bimodal particle diameter distribution in the expanded state, the bimodal distribution having a first mean diameter (D_{1E}) and a second mean diameter (D_{2E}), the ratio of D_{1E}/D_{1C} and D_{2E}/D_{2C} being greater than about 1.5, the ratio of D_{1E}/D_{2E} being about equal to the ratio D_{1C}/D_{2C} with D_{2E} being less than about 10% of the diameter (D_{Vessel}) of the body vessel; and
arranged for occluding the flow of body fluid through the body vessel by the expanded state of the mixture having a particle packing fraction in the body vessel that is at least about 0.85, preferably greater than 0.90 and more preferably greater than 0.95.

10. The method of Claim 9, wherein the step of preparing a mixture of embolic particles in a collapsed state uses a shape memory material selected from the group of polyvinyl alcohol, polyurethane, polymethylmethacrylate, poly(vinyl chloride), polyethylene, metals, metal alloys, and mixtures and combinations thereof.

11. The method of Claim 9, wherein the step of preparing a mixture of embolic particles in the collapsed state has a particle diameter distribution that is tri-modal, the mixture having a third mean diameter (D_{3C}), the ratio of D_{2C}/D_{3C} being less than about 1/7, preferably
wherein the transition from the collapsed state to an expanded state results in the particle diameter distribution remaining tri-modal in the expanded state, the mixture having a third mean diameter (D_{3E}), and the ratio of D_{2E}/D_{3E} being less than about 1/7 with D_{3E} being less than about 10% the diameter of the body vessel (D_{Vessel}).

12. The method of Claim 9, wherein the step of preparing a mixture of embolic particles in the collapsed state includes a fourth mean particle diameter (D_{4C}) in the mixture in its collapsed state, the ratio of D_{3C}/D_{4C} being less than about 1/7.

13. The method of Claim 12, wherein the transition from the collapsed state to an expanded state results in the mixture maintaining a fourth mean diameter (D_{4E}) in the expanded state, the ratio of D_{3E}/D_{4E} being less than about 1/7 with D_{4E} being less than about 10% the diameter of the body vessel (D_{Vessel}).

14. The method of Claim 9, wherein the step of preparing a mixture of embolic particles in the collapsed state further includes particles whose diameter in the collapsed state is substantially the same as their diameter in an expanded state.

15. The method of Claim 9, wherein the method further comprises the step of vibrating the embolic particles during the introduction of the particles to the targeted site of the body vessel or immediately thereafter in order to enhance the packing of the particles;
wherein the vibratory motion arises from moving the catheter or guide wire back and forth in a proximal-distal direction or in a side to side direction.

## Patentansprüche

1. Embolisationssystem mit einer hohen Packungsdichte von embolischen Partikeln zur Verwendung bei der Blockierung des Flusses von Körperflüssigkeit durch ein Körpergefäß, wobei das Embolisationssystem Folgendes umfasst:
ein Gemisch von embolischen Partikeln, die aus einem Formgedächtnismaterial bestehen, wobei die embolischen Partikel einen kollabierten Zustand und einen expandierten Zustand aufweisen und vorzugsweise kugelförmig sind;
wobei das Gemisch zumindest eine bimodale Partikeldurchmesserverteilung im kollabierten Zustand aufweist, wobei die bimodale Verteilung einen ersten mittleren Durchmesser (D_{1C}) und einen zweiten mittleren Durchmesser (D_{2C}) aufweist, wobei das Verhältnis von D_{1C}/D_{2C} kleiner als ca. 1/7 ist;
wobei das Gemisch zumindest eine bimodale Partikeldurchmesserverteilung im expandierten Zustand aufrechterhält, wobei die bimodale Verteilung einen ersten mittleren Durchmesser (D_{1E}) und einen zweiten mittleren Durchmesser (D_{2E}) aufweist, wobei das Verhältnis von D_{1E}/D_{2E} kleiner als ca. 1/7 ist;
wobei das Gemisch so angepasst ist, dass das Verhältnis von D_{1E}/D_{1C} und D_{2E}/D_{2C} größer als ca. 1,5 ist, wobei der expandierte Zustand des Gemisches eine Partikelpackungsdichte im Körpergefäß aufweist, die zumindest ca. 0,85 beträgt;
worin das Gemisch von embolischen Partikeln im kollabierten Zustand in das Körpergefäß abgegeben wird, wobei die Partikelpackungsdichte des Gemisches in seinem expandierten Zustand zur Blockierung des Flusses von Körperflüssigkeit durch das Körpergefäß führt.

2. Embolisationssystem nach Anspruch 1, worin das Formgedächtnismaterial ein aus der aus Polyvinylalkohol, Polyurethan, Polymethylmethacrylat, Poly(vinylchlorid), Polyethylen, Metallen, Metalllegierungen und Gemischen und Kombinationen davon bestehenden Gruppe ausgewähltes Material ist.

3. Embolisationssystem nach Anspruch 1, worin die Partikeldurchmesserverteilung im kollabierten Zustand trimodal ist, wobei das Gemisch einen dritten mittleren Durchmesser (D_{3C}) aufweist, wobei das Verhältnis von D_{2C}/D_{3C} kleiner als ca. 1/7 ist;
wobei die Partikeldurchmesserverteilung im expandierten Zustand trimodal bleibt und einen dritten mittleren Durchmesser (D_{3E}) aufweist, wobei das Verhältnis von D_{2E}/D_{3E} kleiner als ca. 1/7 ist und die Partikelpackungsdichte des Gemisches im expandierten Zustand größer als ca. 0,90 ist.

4. Embolisationssystem nach Anspruch 3, worin das Gemisch ferner einen vierten mittleren Teilchendurchmesser (D_{4C}) im kollabierten Zustand aufweist, wobei das Verhältnis von D_{3C}/D_{4C} kleiner als ca. 1/7 ist;
wobei das Gemisch im expandierten Zustand einen vierten mittleren Durchmesser (D_{4E}) aufweist, wobei das Verhältnis von D_{3E}/D_{4E} kleiner als ca. 1/7 ist und die Partikelpackungsdichte des Gemisches im expandierten Zustand größer als ca. 0,95 ist.

5. Embolisationssystem nach Anspruch 1, worin der mittlere Partikeldurchmesser der embolischen Partikel im expandierten Zustand weniger als ca. 10% des Durchmessers des Körpergefäßes beträgt.

6. Embolisationssystem nach Anspruch 1, worin die Partikeldurchmesserverteilung im kollabierten Zustand trimodal ist, wobei das Gemisch einen dritten mittleren Durchmesser (D₃) aufweist, wobei das Verhältnis von D_{2C}/D₃ kleiner als ca. 1/7 ist;
wobei die Partikeldurchmesserverteilung im expandierten Zustand trimodal bleibt und der dritte mittlere Durchmesser (D₃) gegenüber seinem im kollabierten Zustand gezeigten Durchmesser unverändert bleibt, wobei die Partikelpackungsdichte des Gemisches im expandierten Zustand mehr als ca. 0,85 beträgt.

7. Embolisationssystem nach Anspruch 3, worin das Gemisch ferner im kollabierten Zustand einen vierten mittleren Partikeldurchmesser (D₄) aufweist, wobei das Verhältnis von D₃/D₄ kleiner als ca. 1/7 ist;
wobei das Gemisch im expandierten Zustand einen vierten mittleren Durchmesser (D₄) aufrechterhält, der gegenüber seinem im kollabierten Zustand gezeigten Durchmesser unverändert ist, wobei die Partikelpackungsdichte des Gemisches im expandierten Zustand größer als ca. 0,90 ist.

8. Embolisationssystem nach Anspruch 1, worin das Embolisationssystem ferner eines aus der aus einem Kontrastmittel, einen röntgenopaken Mittel, einem therapeutischen Mittel, flüssigen embolischen Materialien und Gemischen davon bestehenden Gruppe ausgewähltes umfasst.

9. Verfahren zur Bereitstellung eines Embolisationssystems zur Blockierung des Flusses von Körperflüssigkeit durch ein Körpergefäß eines Patienten, wobei das Verfahren Folgendes umfasst:
Zubereitung eines Gemisches von embolischen Partikeln, die aus einem Formgedächtnismaterial bestehen und einen kollabierten Zustand und einen expandierten Zustand aufweisen und vorzugsweise kugelförmig sind, wobei das Gemisch zumindest eine bimodale Partikeldurchmesserverteilung im kollabierten Zustand aufweist, wobei die bimodale Verteilung einen ersten mittleren Durchmesser (D_{1C}) und einen zweiten mittleren Durchmesser (D_{2C}) aufweist, wobei das Verhältnis von D_{1C}/D_{2C} kleiner als ca. 1/7 ist;
wobei das Gemisch von embolischen Partikeln:
zur Abgabe an eine Zielstelle in dem Körpergefäß des Patienten ausgelegt ist;
konfiguriert ist, von dem kollabierten Zustand in einen expandierten Zustand überzugehen, wobei das Gemisch zumindest eine bimodale Partikeldurchmesserverteilung im expandierten Zustand aufrechterhält, wobei die bimodale Verteilung einen ersten mittleren Durchmesser (D_{1E}) und einen zweiten mittleren Durchmesser (D_{2E}) aufweist, wobei das Verhältnis von D_{1E}/D_{1C} und D_{2E}/D_{2C} größer als ca. 1,5 ist, wobei das Verhältnis von D_{1E}/D_{2E} ungefähr dem Verhältnis von D_{1C}/D_{2C} entspricht, wobei D_{2E} weniger als ca. 10% des Durchmessers (D_{Gefäß}) des Körpergefäßes beträgt; und
zur Blockierung des Flusses von Körperflüssigkeit durch das Körpergefäß angeordnet ist, indem das Gemisch im expandierten Zustand eine Partikelpackungsdichte im Körpergefäß aufweist, die zumindest ca. 0,85 beträgt, vorzugsweise größer als 0,90 ist und insbesondere bevorzugt größer als 0,95 ist.

10. Verfahren nach Anspruch 9, worin der Schritt der Zubereitung eines Gemisches aus embolischen Teilchen in einem kollabierten Zustand ein Formgedächtnismaterial verwendet, das aus der aus Polyvinylalkohol, Polyurethan, Polymethylmethacrylat, Poly(vinylchlorid), Polyethylen, Metallen, Metalllegierungen und Gemischen und Kombinationen davon bestehenden Gruppe ausgewählt ist.

11. Verfahren nach Anspruch 9, worin der Schritt der Zubereitung eines Gemisches aus embolischen Teilchen in einem kollabierten Zustand eine trimodale Partikeldurchmesserverteilung aufweist, wobei das Gemisch einen dritten mittleren Durchmesser (D_{3C}) aufweist, wobei das Verhältnis von D_{2C}/D_{3C} kleiner als ca. 1/7 ist;
worin der Übergang von dem kollabierten Zustand in einen expandierten Zustand dazu führt, dass die Partikeldurchmesserverteilung im expandierten Zustand trimodal bleibt, wobei das Gemisch einen dritten mittleren Durchmesser (D_{3E}) aufweist und das Verhältnis von D_{2E}/D_{3E} kleiner als ca. 1/7 ist, wobei D_{3E} weniger als ca. 10% des Durchmessers des Körpergefäßes (D_{Gefäß}) beträgt.

12. Verfahren nach Anspruch 9, worin der Schritt der Zubereitung eines Gemisches aus embolischen Teilchen in einem kollabierten Zustand einen vierten mittleren Partikeldurchmesser (D_{4C}) in dem Gemisch im kollabierten Zustand aufweist, wobei das Verhältnis von D_{3C}/D_{4C} kleiner als ca. 1/7 ist.

13. Verfahren nach Anspruch 12, worin der Übergang von dem kollabierten Zustand in einen expandierten Zustand dazu führt, dass das Gemisch einen vierten mittleren Durchmesser (D_{4E}) im expandierten Zustand aufweist, wobei das Verhältnis von D_{3E}/D_{4E} kleiner als ca. 1/7 ist und D_{4E} weniger als ca. 10% des Durchmessers des Körpergefäßes (D_{Gefäß}) beträgt.

14. Verfahren nach Anspruch 9, worin der Schritt der Zubereitung eines Gemisches aus embolischen Teilchen in einem kollabierten Zustand ferner Partikel aufweist, deren Durchmesser im kollabierten Zustand im Wesentlichen derselbe wie ihr Durchmesser in einem expandierten Zustand ist.

15. Verfahren nach Anspruch 9, worin das Verfahren ferner den Schritt der Vibration der embolischen Partikel bei der Einführung der Partikel an die Zielstelle des Körpergefäßes oder unmittelbar nach zur Erhöhung der Packung der Partikel umfasst,
worin sich die Vibrationsbewegung aus der Vor- und Zurückbewegung des Katheters oder des Führungsdrahts in einer proximal-distalen Richtung oder in einer Seiten-SeitenRichtung ergibt.

## Revendications

1. Système d'embolisation présentant une fraction de bourrage élevée des particules emboliques à utiliser pour occlure l'écoulement d'un fluide corporel à travers un vaisseau corporel, le système d'embolisation comprenant:
le mélange de particules emboliques constituées à partir d'une matière à mémoire de forme, les particules emboliques présentant un état comprimé et un état expansé et étant de préférence de forme sphérique;
le mélange présentant au moins une distribution de diamètre de particule bimodale dans l'état comprimé, la distribution bimodale présentant un premier diamètre moyen (D_{1C}) et un deuxième diamètre moyen (D_{2C}), le rapport D_{1C}/D_{2C} étant inférieur à environ 1/7;
le mélange maintenant au moins une distribution de diamètre de particule bimodale dans l'état expansé, la distribution bimodale présentant un premier diamètre moyen (D_{1E}) et un deuxième diamètre moyen (D_{2E}), le rapport D_{1E}/D_{2E} étant inférieur à environ 1/7;
le mélange étant conçu de telle sorte que les rapports D_{1E}/D_{1C} et D_{2E}/D_{2C} soient supérieurs à environ 1,5, avec l'état expansé du mélange qui présente une fraction de bourrage des particules dans le vaisseau corporel qui est d'au moins environ 0,85, et
dans lequel le mélange de particules emboliques est introduit dans le vaisseau corporel dans l'état comprimé, la fraction de bourrage des particules du mélange dans son état expansé entraînant l'occlusion de l'écoulement de fluide corporel à travers le vaisseau corporel.

2. Système d'embolisation selon la revendication 1, dans lequel la matière à mémoire de forme est sélectionnée dans le groupe comprenant l'alcool polyvinylique, le polyuréthane, le polyméthylméthacrylate, le chlorure de polyvinyle, le polyéthylène, des métaux, des alliages métalliques ainsi que des mélanges et des combinaisons de ceux-ci.

3. Système d'embolisation selon la revendication 1, dans lequel la distribution du diamètre de particule est trimodale dans l'état comprimé, le mélange présentant un troisième diamètre moyen (D_{3C}), le rapport D_{2C}/D_{3C} étant inférieur à environ 1/7; et
la distribution du diamètre de particule restant trimodale dans l'état expansé présentant un troisième diamètre moyen (D_{3E}), le rapport D_{2E}/D_{3E} étant inférieur à environ 1/7, avec la fraction de bourrage des particules du mélange dans l'état expansé qui est supérieure à environ 0,90.

4. Système d'embolisation selon la revendication 3, dans lequel le mélange présente en outre un quatrième diamètre de particule moyen (D_{4C}) dans l'état comprimé, le rapport D_{3C}/D_{4C} étant inférieur à environ 1/7; et
le mélange maintenant un quatrième diamètre moyen (D_{4E}) dans l'état expansé, le rapport D_{3E}/D_{4E} étant inférieur à environ 1/7, avec la fraction de bourrage des particules du mélange dans l'état expansé qui est supérieure à environ 0, 95.

5. Système d'embolisation selon la revendication 1, dans lequel le diamètre moyen de particule des particules emboliques dans l'état expansé est inférieur à environ 10 % du diamètre du vaisseau corporel.

6. Système d'embolisation selon la revendication 1, dans lequel la distribution du diamètre de particule est trimodale dans l'état comprimé, le mélange présentant un troisième diamètre moyen (D₃), le rapport D_{2C}/D₃ étant inférieur à environ 1/7; et
la distribution du diamètre de particule restant trimodale dans l'état expansé, le troisième diamètre moyen (D₃) restant inchangé par rapport à son diamètre affiché dans l'état comprimé, avec la fraction de bourrage des particules du mélange dans l'état expansé qui est supérieure à environ 0,85.

7. Système d'embolisation selon la revendication 3, dans lequel le mélange présente en outre un quatrième diamètre de particule moyen (D₄) dans l'état comprimé, le rapport D₃/D₄ étant inférieur à environ 1/7; et
le mélange maintenant un quatrième diamètre moyen (D₄) dans l'état expansé qui est inchangé par rapport à son diamètre affiché dans l'état comprimé, avec la fraction de bourrage des particules du mélange dans l'état expansé qui est supérieure à environ 0,90.

8. Système d'embolisation selon la revendication 1, dans lequel le système d'embolisation comprend en outre un élément sélectionné dans le groupe comprenant un agent de contraste, un agent radio-opaque, un agent thérapeutique, des matières emboliques liquides ainsi que des mélanges de ceux-ci.

9. Procédé pour fournir un système d'embolisation pour occlure l'écoulement d'un fluide corporel à travers un vaisseau corporel d'un patient, le procédé comprenant les étapes suivantes:
préparer un mélange de particules emboliques constituées à partir d'une matière à mémoire de forme présentant un état comprimé et un état expansé, et étant de préférence de forme sphérique, le mélange présentant au moins une distribution de diamètre de particule bimodale dans l'état comprimé, la distribution bimodale présentant un premier diamètre moyen (D_{1C}) et un deuxième diamètre moyen (D_{2C}), le rapport D_{1C}/D_{2C} étant inférieur à environ 1/7;
ledit mélange de particules emboliques étant:
adapté pour être introduit dans un site cible dans le vaisseau corporel du patient;
configuré pour passer de l'état comprimé à un état expansé, le mélange maintenant au moins une distribution de diamètre de particule bimodale dans l'état expansé, la distribution bimodale présentant un premier diamètre moyen (D_{1E}) et un deuxième diamètre moyen (D_{2E}), les rapports D_{1E}/D_{1C} et D_{2E}/D_{2C} étant supérieurs à environ 1,5, le rapport D_{1E}/D_{2E} étant approximativement égal au rapport D_{1C}/D_{2C}, avec D_{2E} qui est inférieur à environ 10 % du diamètre (Dᵥₐᵢₛₛₑₐᵤ) du vaisseau corporel; et
conçu pour occlure l'écoulement de fluide corporel à travers le vaisseau corporel par l'état expansé du mélange présentant une fraction de bourrage des particules dans le vaisseau corporel qui est d'au moins 0,85, de préférence supérieure à 0,90, et mieux encore supérieure à 0,95.

10. Procédé selon la revendication 9, dans lequel l'étape de préparation d'un mélange de particules emboliques dans un état comprimé utilise une matière à mémoire de forme sélectionnée dans le groupe comprenant l'alcool polyvinylique, le polyuréthane, le polyméthylméthacrylate, le chlorure de polyvinyle, le polyéthylène, des métaux, des alliages métalliques ainsi que des mélanges et des combinaisons de ceux-ci.

11. Procédé selon la revendication 9, dans lequel l'étape de préparation d'un mélange de particules emboliques dans l'état comprimé présente une distribution du diamètre de particule qui est trimodale, le mélange présentant un troisième diamètre moyen (D_{3C}), le rapport D_{2C}/D_{3C} étant inférieur à environ 1/7; et de préférence
dans lequel la transition de l'état comprimé à un état expansé résulte en une distribution de diamètre de particule qui reste trimodale dans l'état expansé, le mélange présentant un troisième diamètre moyen (D_{3E}), le rapport D_{2E}/D_{3E} étant inférieur à environ 1/7, avec D_{3E} qui est inférieur à environ 10 % du diamètre du vaisseau corporel (Dᵥₐᵢₛₛₑₐᵤ).

12. Procédé selon la revendication 9, dans lequel l'étape de préparation d'un mélange de particules emboliques dans l'état comprimé présente un quatrième diamètre de particule moyen (D_{4C}) dans le mélange dans son état comprimé, le rapport D_{3C}/D_{4C} étant inférieur à environ 1/7.

13. Procédé selon la revendication 12, dans lequel il résulte de la transition de l'état comprimé à un état expansé que le mélange maintient un quatrième diamètre moyen (D_{4E}) dans l'état expansé, le rapport D_{3E}/D_{4E} étant inférieur à environ 1/7, avec D_{4E} qui est inférieur à environ 10 % du diamètre du vaisseau corporel (Dᵥₐᵢₛₛₑₐᵤ).

14. Procédé selon la revendication 9, dans lequel l'étape de préparation d'un mélange de particules emboliques dans l'état comprimé comprend en outre des particules dont le diamètre dans l'état comprimé est sensiblement identique à leur diamètre dans un état expansé.

15. Procédé selon la revendication 9, dans lequel le procédé comprend en outre l'étape de vibration des particules emboliques pendant l'introduction des particules dans le site cible du vaisseau corporel ou immédiatement après celle-ci dans le but de renforcer le bourrage des particules; et
dans lequel le mouvement vibratoire provient du déplacement du cathéter ou du fil de guidage d'avant en arrière dans une direction proximale - distale ou dans une direction d'un côté à l'autre.
